# EUROPEAN PATENT SPECIFICATION

(11) **EP 1 385 550 B1**
(45) Date of publication and mention of the grant of the patent: **07.10.2009**
(21) Application number: 02726291.4
(22) Date of filing: 10.05.2002
(51) Int. Cl.: A61K 45/06, A61K 31/557, A61P 37/06

(54) **Therapeutic medicaments for inducing tolerance**
Therapeutische Zusammenseztungen zur Toleranzinduktion
Compositions therapeutiques pour l'induction de la tolerance

(30) Priority: 11.05.2001 GB 0111497
(43) Date of publication of application: 04.02.2004
(73) Proprietor: MEDICAL RESEARCH COUNCIL, London W1B 1AL (GB)
(72) Inventor: KELLY, Rodney, William University of Edinburgh, Edinburgh EH3 9ET (GB)
(74) Representative: Miles, John Stephen
(86) International application number: PCT/GB2002/002114
(87) International publication number: WO 2002/092064

(56) References cited:
- EP-A- 1 199 074
- DE-A- 10 064 991
- MORIUCHI, E. ET AL: "Synergistic inhibition of pannus tissue development in vitro by PGE1 plus phosphodiesterase 4 inhibitors" ARTHRITIS AND RHEUMATISM, vol. 41, no. 9, September 1998 (1998-09), page s161 XP009005530
- SOMMER N ET AL: "Therapeutic potential of phosphodiesterase type 4 inhibition in chronic autoimmune demyelinating disease" JOURNAL OF NEUROIMMUNOLOGY, ELSEVIER SCIENCE PUBLISHERS BV, XX, vol. 79, no. 1, October 1997 (1997-10), pages 54-61, XP002113589 ISSN: 0165-5728

## Description

The present invention relates to therapeutic medicaments and uses; in particular it relates to medicaments for inducing tolerance to an antigen in a patient.

An organism's immunity to an antigen arises as a consequence of a first encounter with the antigen and the subsequent production of immunoglobulin molecules, for example, antibodies, capable of selectively binding that antigen. In addition, the immune response is controlled by T cells which may be antigen specific. Immunity allows the rapid recruitment, usually by stimulating an inflammatory response, of cells which can dispose of the foreign antigen. Under certain circumstances, the immune system does not produce an immune response against antigens due to a mechanism called "tolerance". For example, an immune system can normally discriminate against foreign antigens and constituents of the organism itself, due to a mechanism whereby all B lymphocytes which could potentially produce antibodies to constituents of the organism itself ("self antigens") are destroyed during development, thereby removing the organism's capacity to produce antibodies directed to a self antigen.

Tolerance is probably an active process. This means that peripheral tolerance is gained where an antigen is presented to a T cell in a particular environment (eg high IL-10 levels and low IL-12 levels). The T cells then circulate and when they meet that specific antigen again they do not mount an immune response (anergic T cells) or they mount a quelling response (regulatory T cells). A role for regulatory T cells has been proposed in tolerance. The regulatory T cells are programmed by the environment of the antigen presenting cell to react to their cognate antigen by releasing "down-regulatory" cytokines. The first such regulatory cells described were induced by IL-10 (Groux et al., 1997, Nature 389:737-742).

Where tolerance breaks down, the organism may produce a cellular immune response (including cytotoxic T cells) to normal constituents of the organism, producing an "autoimmune disease". Examples of autoimmune diseases include systemic lupus erythematosus (SLE), multiple sclerosis (MS) and Hashimoto's disease.

In some circumstances, even the normal response of the immune system to a foreign antigen can produce undesirable results, such as in the case of tissue or organ grafts or transplants, where the immune system of the tissue or organ recipient recognises the tissue or organ graft or transplant as foreign and acts to reject it

One of the drawbacks of existing methods of treating immune or inflammatory conditions or diseases however, is the limited range of options and their therapeutic inadequacy. For example, glucocorticosteroids used for treating inflammatory respiratory disease have toxic effects in many patients, and alternatives such as cyolosporin A or interferon γ are high-risk, expensive and generally unsatisfactory.

Unexpectedly, the inventor has found that there is a synergistic effect between a prostaglandin that stimulates cAMP production in macrophages and a type IV selective phosphodiesterase (PDE) inhibitor on the release of interleukin-10 (IL-10) from cells of the immune system. Furthermore, the inventor has found that there is a marked stimulation of IL-10 and inhibition of interleukin-12 (IL-12) in cells of the immune system when a prostaglandin that stimulates cAMP production in macrophages and a type IV selective PDE inhibitor are used in combination. In the presence of a type IV selective PDE inhibitor, the stimulation of IL-10 by both PGE and 19-hydroxy PGE was increased strikingly.

Type IV selective PDE inhibitors such as Rolipram are known to raise cAMP and IL-10 levels in monocyte/macrophages stimulated with the bacterial coat product lipopolysaccharide (LPS) (Strassman et al., 1994 J. Exp. Med. 180: 2365-70; Kraan et al., 1995 J. Exp. Med. 181: 775-9; Kambayashi et al., 1995 J. Immunol. 155: 4909-16). Unexpectedly, the inventor has found that there is a synergistic effect between a prostaglandin that stimulates cAMP production in macrophages and a type IV selective PDE inhibitor on the release of IL-10 from cells of the immune system, which results in a dramatic increase in the release of IL-10.

The inventor also shows an increase in PDE activity that follows both PGE and 19-hydroxy PGE application. This is a direct negative feedback to reduce the effect of the stimulus. Use of a PGE and a type IV elective PDE inhibitor increases PDE message even further, but then the synthesised phosphodiesterase is nullified by the presence of the inhibitor.

In diseases resulting from an aberrant or undesired immune response there is often a deficiency in IL-10 and/or an increase in IL-12. This imbalance in IL-10 may be detrimental to the development of useful T helper cells, particularly T regulatory cells; a preponderance of type 1 T helper cells over type 2 T helper cells is thought to be characteristic of autoimmune disease. Thus, stimulation of IL-10 production and inhibition of IL-12 is believed to induce a tolerising environment for T cell activation.

The inventor now proposes the use of a type IV selective PDE inhibitor in combination with a prostaglandin or agonist thereof that stimulates cAMP production in macrophages in the induction of tolerance of, or tolerance to, an antigen in a patient.

Furthermore, the combination of a type IV selective PDE inhibitor and a prostaglandin or agonist thereof that stimulates cAMP production in macrophages is considered by the inventor to achieve the desirable effect of reducing the amount of prostaglandin or agonist thereof that stimulates cAMP production in macrophages or PDE inhibitor required to achieve a useful degree of therapeutic benefit, and/or reducing the side effects of administration of prostaglandin or agonist thereof that stimulates cAMP production in macrophages.

As far as the inventor is aware, there has never been any suggestion that a combination of a prostaglandin or agonist thereof that stimulates cAMP production in macrophages and a type IV selective inhibitor of PDE could be used to stimulate IL-10 production, and there has been no suggestion of a treatment using this combination to stimulate IL-10. Furthermore, there has never been any suggestion that this combination could be used to inhibit IL-12 production, or to induce a tolerising environment for T cell activation, or to induce tolerance to an antigen in a patient.

The use of a combination of a prostaglandin and a PDE inhibitor to alleviate the symptoms of psoriasis and related proliferative skin disorders has been suggested in US 4,034,087, without actually providing any examples of a prostaglandin and a PDE inhibitor being used to treat them. Such an application of a PGE and PDE inhibitor is anti-inflammatory rather than immunomodulatory. Moriuchi et al., 1998 arthritis and rheumatism 41(9) S161 discloses the use of pentoxyfylline or rolipram in combination with pge1 in the treatment of rheumatoid arthritis.

The principal receptor for prostaglandin E2 (PGE2) are the EP₂ and EP₄ sub-types, however other receptor sub-types exist, namely EP₁ and EP₃. EP₂ and EP₄ receptors couple with adenylcyclase and use elevated cAMP as the messenger system. The levels of cAMP in tissue are governed both by synthesis and by catabolism by PDE. PDE can be blocked by specific inhibitors. The inventor believes, but without being bound by any theory, that the administration of a type IV selective PDE inhibitor will enhance the effect of a prostaglandin or agonist thereof that stimulates cAMP production in macrophages in inducing tolerance to an antigen in a patient. Thus, the inventor believes, but without being bound by any theory that the effect of a prostaglandin or agonist thereof that stimulates cAMP production in macrophages (such as PGE) acting on its EP₂ and EP₄ receptors is to stimulate cAMP and the addition of the type IV selective PDE inhibitor provides a synergistic action on monocytes and macrophages resulting in a reduction in the immune response which is greater than the effect of the sum of the same amount of either prostaglandin or agonist thereof that stimulates cAMP production in macrophages or type IV selective PDE inhibitor administered alone.

A first aspect of the invention provides the use of a prostaglandin or agonist thereof, that stimulates cAMP production in macrophages or a type IV selective PDE inhibitor, or a combination thereof in the manufacture of a medicament for inducing tolerance to an antigen in a patient, as set out in claims 1 to 3.

By inducing tolerance to an antigen we include the meaning that the immune system of the patient may become tolerant of an antigen where it was intolerant before, or the immune system may mount a reduced response or no response at all (*ie*, an undetectable response) to an immune stimulus such as an antigen.

An effect of the treatment of a patient with a prostaglandin or agonist thereof that stimulates cAMP production in macrophages and a type IV selective PDB inhibitor may be the facilitation or improvement of tolerance to an antigen. The antigen may be one which is foreign to the patient, such as an antigen which is involved in irritable bowel syndrome.

It will also be appreciated that the induction of tolerance to an antigen in a patient upon administration of prostaglandin or agonist thereof that stimulates cAMP production in macrophages and a type IV selective PDE inhibitor may lead to antigen specific immune suppression. Thus, the invention includes the manufacture of a medicament for inducing tolerance to an antigen in a patient to create a state of immune suppression in the patient, comprising a prostaglandin or agonist thereof that stimulates cAMP production in macrophages or a type IV selective PDE inhibitor or combination, thereof. Such a state of immune suppression is characterised by raising the threshold of a cell-mediated immune response to any antigenic stimulus.

Thus, it will be seen that the invention also provides the use of the combination of a prostaglandin or agonist thereof that stimulates cAMP production in macrophages and a PDE inhibitor in the manufacture of an immunosuppressant.

The invention therefore includes the manufacture of medicaments for suppressing the immune system in a patient. By "suppressing" we include the meaning that the immune system response is altered such the immune system mounts a reduced response or no response to an immune stimulus. Accordingly, the invention includes the manufacture of medicament for inducing tolerance to an antigen in a patient leading to amelioration of an aberrant or undesired immune response in the patient.

The medicament manufactured according to the invention also include those that induce tolerance to an antigen in a patient for the treatment of diseases or conditions where there is an undesirable immune response.

The invention includes the manufacture of a medicament for treating an autoimmune disease in a patient, comprising a prostaglandin or agonist thereof that stimulates cAMP production in macrophages or a type IV selective PDE inhibition or combinations thereof. Such autoimmune diseases include primary myxoedema, thyrotoxicosis, pernicious anaemia, autoimmune atrophic gastris, Addison's disease, insulin-dependent diabetes mellitus (IDDM), Goodpasture's syndrome, myasthenia gravis, sympathetic ophthalmia, MS, autoimmune haemolytic anaemia, idiopathic leucopenia, ulcerative colitis, dermatomyositis, scleroderma, mixed connective tissue disease, irritable bowel syndrome, SLE, Hashimoto's disease, thyroiditis, Behcet's disease, coeliac disease/dermatitis herpetiformis, and demyelinating disease.

The invention includes the manufacture of a medicament for treating an autoimmune disease, with the exception of rheumatoid arthritis, in a patient, comprising a prostaglandin or agonist thereof that stimulates cAMP production in macrophages or a type IV selective PDE inhibitor or a combination thereof.

The medicament is believed to combat the undesirable autoimmune response directly, as well as treating the symptoms by directing T cells away from a pro-inflammatory role.

Without being bound by theory, the inventor believes that the medicaments of the present invention may affect the programming of T cells so that they become regulatory or suppressive T cells rather than pro-inflammatory T cells. When a T cell meets an antigen, in the presence of a prostaglandin that stimulates cAMP production in macrophages and type IV selective PDE inhibitor, it will release a suppressive cytokine such as IL-10 and not an inflammatory cytokine such as IL-12. Treatment with a prostaglandin that stimulates cAMP production in macrophages and type IV selective PDE inhibitor is thus believed to prevent or minimise an inflammatory response from developing. Thus treatment with a prostaglandin that stimulates cAMP production in macrophages and type IV selective PDE inhibitor can be used prophylactically, or as soon as the first symptoms of, *eg* an autoimmune disease, appear. Furthermore, it will be appreciated that because T cells are present throughout the body they may be programmed or primed at a site remote from their ultimate site of action. Similarly, unlike other forms of treatment of certain autoimmune diseases, the medicament may be helpful in preventing inflammatory responses before they start. Thus, the medicament may be useful in treating patients who, for example because of their age or genetic factors, are predisposed to an autoimmune disease before any inflammatory symptoms show.

Thus, the invention also includes the manufacture of a medicament for inducing tolerance to an antigen in a patient for inhibiting or dampening an immune or inflammatory response in the patient. By "inhibition or dampening" we include increasing the level of IL-10, and/or decreasing the level of IL-12 which leads to an increase in the Th2 response a decrease in the Th1 response

The invention also includes the manufacture of a medicament for inducing tolerance to an antigen in a patient leading to amelioration of an aberrant or undesired inflammatory response in the patient. The aberrant or undesired inflammator response is not associated with psoriasis, or a related proliferative skin disorder.

By "aberrant or undesired immune or inflammatory response" we include diseases or conditions which cause the presence of visible or measurable inflammation within a tissue in an individual or patient. For example, the tissue that forms part of an allograft or the tissues of a host having received an allograft, or the central nervous system of an individual with MS, or insulitis in a patient with type 1 diabetes.

The invention includes the manufacture of a medicament for inducing tolerance to an antigen in a patient thereby suppressing an aberrant or undesired immune or inflammatory response in the patient, such as a response related to transplant rejection.

The invention therefore includes the manufacture of a medicament for the treatment of a disease or condition associated with transplant rejection such as graft versus host disease or host versus graft disease, for example in organ or skin transplants. In these cases, an inhibition or dampening of an immune or inflammatory response may be required. Thus, the invention includes the combating of transplant rejection.

Diseases or conditions where there is an aberrant or undesired immune or inflammatory response may also include allergies, wherein the undesired response is an allergic response. In such a condition or disease, the antigen to which tolerance is induced would be an allergen.

Thus, the invention includes the manufacture of a medicament for treating an allergic condition or disease in a patient, comprising a prostaglandin or agonist thereof that stimulates cAMP production in macrophages or a type IV selective PDE inhibitor or a combination thereof.

The allergy may be any allergy such as allergy to cat dander, house dust mite, grass or tree pollens, fungi, moulds, foods, stinging insects and so on.

In one preferred embodiment, the allergic condition or disease is allergic asthma, and the PG that stimulates cAMP production in macrophages and the type IV selective PDE inhibitor are preferably administered to the lungs or bronchial tree, preferably *via* an aerosol. This embodiment may be particularly advantageous as some 19-hydroxy prostaglandin analogues have been reported to function as bronchodilators, such as those described in US Patent No. 4,127,612. The reason why PGs are not widely used in the treatment of asthma is that they make the patient cough. Administration of a type IV selective PDE inhibitor would allow the PG that stimulates cAMP production in macrophages to be administered at a lower concentration thus providing the therapeutic benefits while minimising the side-effects.

Thus the invention includes the use of a PGE or 19-hydroxy PGE and a type IV selective PDE inhibitor for in the manufacture of a medicament for the treatment by inhalation of asthma due to allergy.

Whether or not a particular patient is one who is expected to benefit from treatment may be determined by the physician.

The prostaglandin or agonist thereof that stimulates cAMP production in macrophages and the type IV selective PDE inhibitor may be administered in any order. Preferably, they are co-administered. However, they may be administered so that the type IV selective PDE inhibitor can take effect in the accessory cells prior to administration of the prostaglandin, or agonist thereof that stimulates cAMP production in macrophages. The prostaglandin or agonist thereof that stimulates cAMP production in macrophages and the type IV selective PDE inhibitor may be administered substantially simultaneously, for example in the same composition. The order and timing of administration may be determined by the physician using knowledge of the properties of the prostaglandin that stimulates cAMP production in macrophages and type IV selective PDE inhibitor. For example, the prostaglandin that stimulates cAMP production in macrophages (such as misoprostol) may be active over a period of 4 hours following administration. The type IV selective PDE inhibitor may take of the order of 30 minutes to take effect after administration. Thus, suitable timings of administration can readily be devised from this information.

Where the tolerance to an antigen is desired to be localised to a particular organ, for example to the skin or the bronchial tree and lungs, it is preferred if the prostaglandin or agonist thereof that stimulates cAMP production in macrophages is administered locally at the site of the condition. The prostaglandin or agonist thereof that stimulates cAMP production in macrophages may be administered as a gel or cream or vapour or spray or in a "patch"' in the case of a condition localised to the skin, or as an inhaled vapour or spray where the site is the lungs or bronchial tree.

As is described in more detail below, the prostaglandin or agonist thereof that stimulates cAMP production in macrophages may be administered systemically, such as orally. For example, the prostaglandin or agonist thereof that stimulates cAMP production in macrophages may be administered to the mucosal immune system, *eg via* a suppository, and is expected to act at mucosal sites remote from administration.

The type IV selective PDE inhibitor may be administered by any suitable route. The type IV selective PDE inhibitor may reach the desired site of inhibition of type IV PDE, which is typically the leukocytes in relation to the present invention using many different routes of administration. Typically, in one embodiment, the type IV selective PDE inhibitor is administered systemically. Suitable forms of systemic administration include oral, transcutaneous or by suppository. The type IV selective PDE inhibitor may be administered to the mucosal immune system, *eg via* a suppository, and is expected to act at mucosal sites remote from administration. Type IV selective PDE inhibitors are orally available, so it may be convenient to administer the type IV selective PDE inhibitor orally.

It is also convenient to administer the type IV selective PDE inhibitor locally. Thus, the type IV selective PDE inhibitor may be delivered locally, such as on the skin, using, for example, a gel or cream or vapour or spray or in a "patch" as described above in relation to the administration of the prostaglandin or agonist thereof that stimulates cAMP production in macrophages. Similarly, in the case of administration to the bronchial tree or lungs it may be administered as a spray or vapour.

In preferred embodiments of the invention the prostaglandin or agonist thereof that stimulates cAMP production in macrophages and the type IV selective PDE inhibitor may be combined in the same formulation for delivery simultaneously. Thus, the prostaglandin or agonist thereof that stimulates cAMP production in macrophages and the PDE inhibitor may be combined in a gel or a cream or a vapour or spray or "patch" or suppository and administered together to the patient

In a preferred embodiment, a suppository containing PGE or 19 hydroxy PGE and a type IV selective PDE inhibitor has an enteric coating which only releases the active agents in the bowel when the pH has risen. This sort of preparation has been successful in the delivery of glucocorticoids to the bowel (data sheet for Entocort CR).

Alternatively, the PG that stimulates cAMP production in macrophages and PDE inhibitor can be administered in a capsule or other suitable form that is swallowed. The capsule or other suitable has an enteric coating which is pH sensitive leading to release at an appropriate point in the gastrointestinal tract where it is desired to do so, typically the distal ileum or colon.

Alternatively the prostaglandin that stimulates cAMP production in macrophages and/or type IV selective PDE inhibitor may be administered directly to the colon or distal ileum *via* a non-soluble tube or pipe system, such as produced by Egalet.

A suppository containing PGE or 19 hydroxy PGE and a type IV selective PDE inhibitor may be effective for treating inflammatory bowel disease, which can be caused by antigen-specific immune responses (Groux *et al*).

The administration of prostaglandin that stimulates cAMP production in macrophages and type IV selective PDE inhibitor to a mucosal site remote from the site of inflammation, *eg* co-administration as a suppository in the treatment of arthritis, may be particularly advantageous as pathologic changes in the gastrointestinal tract can be associated with clinical complaints in multiple organs, including the musculoskeletal system (Alghafeer er & Sigal, Bulletin on the Rheumatic Diseases, 51(2): http://www.arthritis.org/research/bulletin/vol51no2/51_2printable.asp). Some reactive arthritis can be triggered by mflammatory bowel diseases, and lymphocytes from the gut mucosa have been reported to migrate to joint tissue in enteropathic arthritis (Salmi & Jalkanen (2001) J Immunol.,166(7): 4650-7).

The prostaglandin or agonist thereof that stimulates cAMP production in macrophages may be any suitable prostaglandin or agonist thereof that stimulates cAMP production in macrophages. By "prostaglandin or agonist" we mean any compound which acts as a prostaglandin agonist that stimulates cAMP production in macrophages on a prostaglandin receptor. The prostaglandin agonist that stimulates cAMP production in macrophages need not be a prostanoid. Typically, the agonist is one which binds the EP₂ or EP₄ receptor. It is preferred that the prostaglandin that stimulates cAMP production in macrophages is a PGE or a PGL Preferably, the prostaglandin that stimulates cAMP production in macrophages is not a PGF or agonist thereof. It is preferred that the prostaglandin or agonist thereof that stimulates cAMP production in macrophages is is PGE₂ or a synthetic analogue thereof. Synthetic analogues include those modified at position 15 or 16 by the addition of a methyl group or those where the hydroxyl has been transposed from position 15 to position 16. Preferred examples of analogues of a prostaglandin that stimulates cAMP production in macrophages include Butaprost (an EP₂ receptor agonist) and 11-deoxy PGE1 (an EP₄ receptor agonist). For the avoidance of doubt, the term "prostaglandin" includes a naturally-occurring prostaglandin that stimulates cAMP production in macrophages as well as a synthetic prostaglandin analogue that stimulates cAMP production in macrophages.

A suitable prostaglandin or agonist thereof that stimulates cAMP production in macrophages includes dinoprostone (sold as Propess^{™} by Ferring in Europe and Forest in the USA; sold as Prostin E2^{™} by Phamacia), gemeprost (sold by Farillon), misoprostol (which is sold as Cytotec^{™} by Searle and Pharmacia), alprostadil (which is sold as Caverject^{™} by Pharmacia and Viridal^{™} by Schwarz and MUSE^{™} by AstraZeneca) and limaprost.

Misoprostol is a PGE analogue which has EP2 and EP3 agonist effects. Its chemical stricture is (±) methyl 11α, 16-dihydroxy-16-methyl-9-oxoprost-13-enoate.

An example of a non-prostanoid compound which acts as a prostaglandin agonist that stimulates cAMP production in macrophages is AH23848, an EP4 receptor agonist.

EP2 agonists which may be useful in the practise of the invention include AH13205.

A suitable prostaglandin thereof that stimulates cAMP production in macrophages also include 19-hydroxy PGE1 and 19-hydroxy PGE2. Prostaglandin agonist are described in EP 1 097 922 and EP 1114 816.

A suitable prostaglandin or agonist thereof that stimulates cAMP production in macrophages may also include any of the 19-hydroxy prostaglandin analogues described in US Patent No. 4,127,612.

It is preferred that the prostaglandin that stimulates cAMP production in macrophages is prostaglandin E₂ (PGE₂). A prostaglandin and agonist thereof that stimulates cAMP production in macrophages including PGE₂, is commercially available, for example from Pharmacia and Upjohn as Prostin E2™.

The type IV selective PDE inhibitor may be any suitable type IV selective PDE inhibitor, Preferably, the type IV selective PDE inhibitor inhibits type IV PDEs which are known to be active in cAMP breakdown. By "selective" we mean that the inhibitor inhibits type IV PDE more potently than another type. Preferably, the type IV selective inhibitor is at least 2 times more potent an inhibitor of type IV PDE than another PDE type. More preferably, the type IV selective inhibitor is at least 5 times, 10 times, 20 times, 30 times, 40 times, 50 times, 100 times, 200 times, 500 times or 1000 times more potent an inhibitor of type IV PDB than another PDE type. Typically, the inhibitor is around 5 to 50 times more potent an inhibitor of the PDE type IV than another PDE type. Typically, the inhibitor is 5 to 50 times more potent an inhibitor of type IV PDE than an inhibitor that is considered to be non-selective such as theophylline. Thus, theophylline is 30 times less effective than rolipram.

Preferably, selective inhibition is determined by a comparison of IC₅₀ levels (Dousa (1999) Kidney International 55: 29-62).

US Patent No. 6,127,378 discloses phenanthridines substituted in the 6 position that are described as elective PDE inhibitors (mainly of type IV), that may be suitable for use in the manufacture of the medicaments of the invention.

Specific (or selective) type IV PDE inhibitors include rolipram (4-[3-cyclopentyloxy-4-methoxyphenyl]-2-pyrrolidinone) and Ro-20-1724 (4-[3-butoxy-4-methoxybenzyl]-2-imidazolidinone). The IC₅₀ for rolipram is 800nM, and the IC₅₀ for Ro-20-1724 is 2 µM.

Another suitable PDE type IV selective inhibitor is denbufylline (1,3-di-n-butyl-7-(2-oxopropyl)-xanthine).

CP 80 633 (Hanifin et al (1996) J. Invest. Dermatol. 107, 51-56), CP 102 995 and CP 76 593 are also all potent type IV inhibitors (available from Central Research Division, Pfizer Inc, Groton, CT).

Other high affinity type IV selective PDE inhibitors include CPD 840, RP 73401, and RS 33793 (Dousa, 1999). The high affinity type IV selective PDE inhibitors have a Kᵢ of approximately 1 nM while the lower affinity inhibitors have a Kᵢ of about 1 µM.

The articles by Dousa (1999); Müller et al (1996, Trends Pharmacol. Sci. 17: 294-298); Palfreyman & Souness (1996, Prog Med Chem 33: 1-52)_{;} Stafford & Feldman (1996, Annual Reports in Medicinal Chemistry (vol 31) pp 71-80; Ed. Bristol, Academic Press, NY, USA); and Teixeira et al (1997, Trends Pharmacol. Sci. 18: 164-171) all contain disclosures relating to type IV PDE selective inhibitors.

Typically, when a type IV PDE-selective inhibitor is administered orally, around 1 to 30 mg is used. Thus, a typical oral dose of rolipram or denbufylline is 1 mg or 5 mg or 5 mg or 10 mg or 30 mg.

In one embodiment the prostaglandin or agonist thereof that stimulates cAMP production in macrophages is administered orally. In particular the prostaglandin or agonist thereof that stimulates cAMP production in macrophages is a prostaglandin analogue which has been modified to reduce its catabolism and which is orally available (such as misoprostol).

Although the type IV selective PDE inhibitor can be administered by any suitable means and by any suitable route, when the prostaglandin or agonist thereof that stimulates cAMP production in macrophages is administered orally it is preferred that the type IV selective PDE inhibitor is also administered orally. It is also preferred if the prostaglandin or agonist thereof that stimulates cAMP production in macrophages and type IV selective PDE inhibitor are administered simultaneously, for example in the same composition.

Thus, in a preferred embodiment, the medicament manufactured in accordance with the invention is for the oral administration of a prostaglandin analogue that stimulates cAMP production in macrophages which has been modified to reduce its catabolism and which is orally available (such as misoprostol) and the oral administration of the type IV selective PDE inhibitor, such as rolipram. The advantages of oral administration is that it generally has good compliance compared to other modes of administration.

The inventor believes that the combination of type IV selective PDE inhibitor with the orally available prostaglandin or agonist thereof that stimulates cAMP production in macrophages will mean that a lower dose of oral prostaglandin will be required than in the absence of the type IV selective PDE inhibitor. It is believed by the inventor that this will have the advantage of reducing side effects caused by the oral prostaglandin or agonist thereof that stimulates cAMP production in macrophages such as muscle cramps.

Typically, 0.1 - 100 µg of 19 hydroxy PGE and 1 -250 µg Rolipram in 5 ml saline would be administered.

Topically, 100 to 800 µg of misoprostol is administered orally daily with 1 to 30 mg of rolipram or denbufylline.

As described above, the prostaglandin or agonist thereof that stimulates cAMP production in macrophages can be used orally in combination with a PDE inhibitor at a lower dose than in the absence of PDE inhibitor.

Typically, the dose of type IV selective PDE inhibitor is as described above and the prostaglandin that stimulates cAMP production in macrophages, such as misoprostol, is administered at a dose of 100 to 400 µg.

The data described in the Figures and Examples shows that typically a higher concentration of 19-hydroxy PGE would be necessary to achieve similar effects to PGE. However, 19 hydroxy PGE has the advantage of being more rapidly catabolised.

Thus, preferably, the combination of a type IV selective PDE inhibitor and prostaglandin or agonist thereof that stimulates cAMP production in macrophages, comprises a selective type IV PDE inhibitor and a 19-hydroxy PGE.

A second aspect of the invention provides the use of a prostaglandin or agonist thereof that stimulates cAMP production in macrophages in the manufacture of a medicament for inducing tolerance to an antigen in a patient wherein the patient, is administered a type IV selective PDE inhibitor and wherein the combination of the prostaglandin or agonist thereof and the type IV selective PDE inhibitor is used to induce tolerance to an antigen in a patient. Thus, the patient may already have been administered the type IV selective PDE inhibitor before administration of the prostaglandin or agonist thereof that stimulates cAMP production in macrophages, or is administered the type IV selective PDE inhibitor at the same time as the prostaglandin or agonist thereof that stimulates cAMP production in macrophages or will be administered the type IV selective PDE inhibitor after administration of the prostaglandin or agonist thereof that stimulates cAMP production in macrophages.

A third aspect of the invention is the use of a type IV selective PDE inhibitor in the manufacture of a medicament for inducing tolerance to an antigen in a patient wherein the patient is administered a prostaglandin or agonist thereof that stimulates cAMP production in macrophages and wherein the combination of the prostaglandin or agonist thereof and the type IV selective PDE intubitor is used to induce tolerance to an antigen in a patient. Thus, the patient may already have been administered the prostaglandin or agonist thereof that stimulates cAMP production in macrophages before administration of the type IV selective PDE inhibitor, or is administered the prostaglandin or agonist thereof that stimulates cAMP production in macrophages at the same time as the type IV selective PDE inhibitor or will be administered the prostaglandin or agonist thereof that stimulates cAMP production in macrophages after administration of the type IV selective PDE inhibitor.

A fourth aspect of the invention provides the use of a combination of a prostaglandin or agonist thereof that stimulates cAMP production in macrophages and a type IV selective PDE inhibitor in the manufacture of a medicament for inducing tolerance to an antigen in a patient. Thus, the prostaglandin or agonist thereof that stimulates cAMP production in macrophages and type IV selective PDE inhibitor may be combined in the same medicament before administration to the patient.

Preferably, the use according to the second, third and fourth aspects is in treating an aberrant or undesired immune or inflammatory response in the patient.

The preferences for the prostaglandin or agonist thereof that stimulates cAMP production in macrophages, type IV selective PDE inhibitors, routes of administration, doses and so on for the second, third and fourth aspects of the invention are the same as for the first aspect of the invention.

A fifth aspect of the invention provides a therapeutic system for use in inducing tolerance to an antigen, the system comprising a prostaglandin or agonist thereof that stimulates cAMP production in macrophages and a type IV selective PDE inhibitor. The therapeutic system may also be termed a "kit of parts".

Preferably, the therapeutic system contains a preferred prostaglandin or agonist thereof that stimulates cAMP production in macrophages as defined in the first aspect of the invention. Still preferably, the therapeutic system contains a preferred type IV selective PDE inhibitor as defined in the first aspect of the invention. The therapeutic system or kit of parts may suitably contain both the prostaglandin or agonist thereof that stimulates cAMP production in macrophages and the type IV selective PDE inhibitor packaged and presented in suitable formulations for use in combination, either for administration simultaneously or for administration which is separated in time. Thus, for example, in one embodiment where the prostaglandin or agonist thereof that stimulates cAMP production in macrophages and type IV selective PDE inhibitor are for simultaneous administration locally to the skin, the therapeutic system may contain a gel or cream or spray or vapour or "patch" which contains a combination of prostaglandin or agonist thereof that stimulates cAMP production in macrophages and PDE inhibitor. Alternatively, in another embodiment where the prostaglandin or agonist thereof that stimulates cAMP production in macrophages and type IV selective PDE inhibitor are for separate administration in a particular treatment regime, the prostaglandin or agonist thereof that stimulates cAMP production in macrophages and PDE inhibitor are packaged or formulated separately. For example, the prostaglandin or agonist thereof that stimulates cAMP production in macrophages may be formulated for administration locally using a cream or gel or spray or vapour or "patch", and the type IV selective PDE inhibitor is packaged or formulated for systemic administration such as oral administration.

The formulations of the prostaglandin or agonist thereof that stimulates cAMP production in macrophages alone or type IV selective PDE inhibitor alone or the combination thereof may conveniently be presented in unit dosage form and may be prepared by any of the methods well known in the art of pharmacy. Such methods include the step of bringing into association the active ingredients used in the invention with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration (eg of the type IV selective PDE inhibitor or of a suitable prostaglandin or agonist thereof that stimulates cAMP production in macrophages) may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent The tablets may optionally be coated or scored and may be formulated so as to provide slow or controlled release of the active ingredient therein using, for example, hydroxypropylmethylcellulose in varying proportions to provide desired release profile.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

For local administration to the skin, it may be convenient to formulate the prostaglandin or agonist thereof that stimulates cAMP production in macrophages and/or type IV selective PDE inhibitor in combination with a dispersion agent or an agent which allows for increased transdermal or transmucosal transfer or penetration, such a dimethyl sulphoxide (DMSO) and the like. Suitable agents are ones which are compatible with the prostaglandin or agonist thereof that stimulates cAMP production in macrophages and/or type IV selective PDE inhibitor (eg are solvents thereof).

A composition comprising a prostaglandin or agonist thereof that stimulates cAMP production in macrophages and a type IV selective PDE inhibitor is useful in the practice of the invention.

Typically, the composition is packaged and presented for use in medicine. The composition may be used in human or veterinary medicine; preferably, it is used in human medicine.

Typically, the composition further comprises a pharmaceutically acceptable carrier. Thus, a pharmaceutical composition (or formulation as it may be termed) comprising a prostaglandin or agonist thereof that stimulates cAMP production in macrophages, a type IV selective PDE inhibitor and a pharmaceutically acceptable carrier is useful in the practice of the invention. The carrier(s) must be "acceptable" in the sense of being compatible with the composition of the invention and not deleterious to the recipients thereof. Typically, the carriers will be water or saline which will be sterile and pyrogen free.

The patient on which the medicament is used is preferably a human although the patient may be any mammal such as a cat, dog, horse, cow, sheep, horse, pig and so on.

It will be appreciated that the medicament may be used before symptoms indicating a need to induce tolerance of an antigen becomes apparent in the patient to be treated, or, either alternatively or in addition, the using of the medicament may be used after symptoms or signs become apparent. Thus, in the case of a patient receiving an organ or tissue transplant, it may be beneficial to administer the prostaglandin or agonist thereof that stimulates cAMP production in macrophages and type IV selective PDE inhibitor before the transplantation surgery is started. It may be further beneficial to continue the administration during or after completion of the transplant or graft surgery. The necessary dosage may be determined by the physician, according to the degree of tolerance that is required.

It will further be appreciated that each of the prostaglandin or agonist thereof that stimulates cAMP production in macrophages, the type IV selective PDE inhibitor may be administered as a single dose, or in multiple smaller doses which achieve the same therapeutic effect. The frequency of administration may vary according to the convenience of the physician administering the dose or the patient.

Pregnancy is likely to be a contraindication for the present invention. In fact, pregnancy is a contraindication for several prostaglandins including misoprostol. Cytotec^{™} (misoprostol) does not cause hypotension, but this may be a possible risk with the method of the invention.

The invention will now be described in more detail with the aid of the following Figures and Examples.

### Figure 1

Expression of mRNA for cytokines IL-10 and IL-12 subunit p35. Experiment carried out on U937 cells (pro-monocytes) in the presence of Rolipram at 1 µg/ml = 4 µM and indomethacin 10 µM. The indomethacin prevents prostaglandin synthesis from cells. Note that the effect of PGE+Rolipram is a marked stimulation of IL-10 and an inhibition of IL-12 both for unstimulated and IFNγ stimulated cells. Vertical scale is a measure of mRNA compared to a control sample as measured by real-time quantitative PCR (Taqman).

### Figure 2

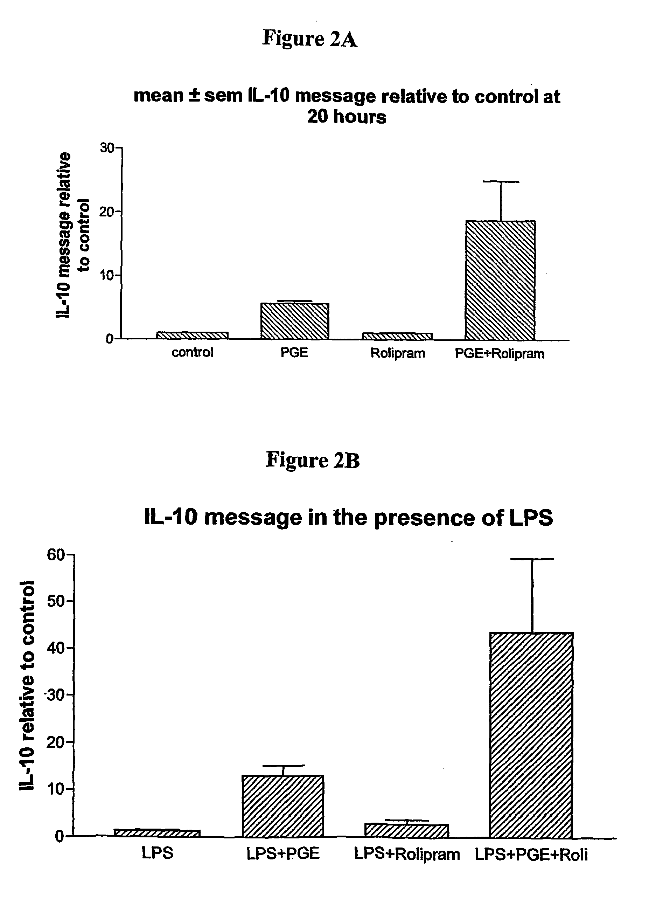

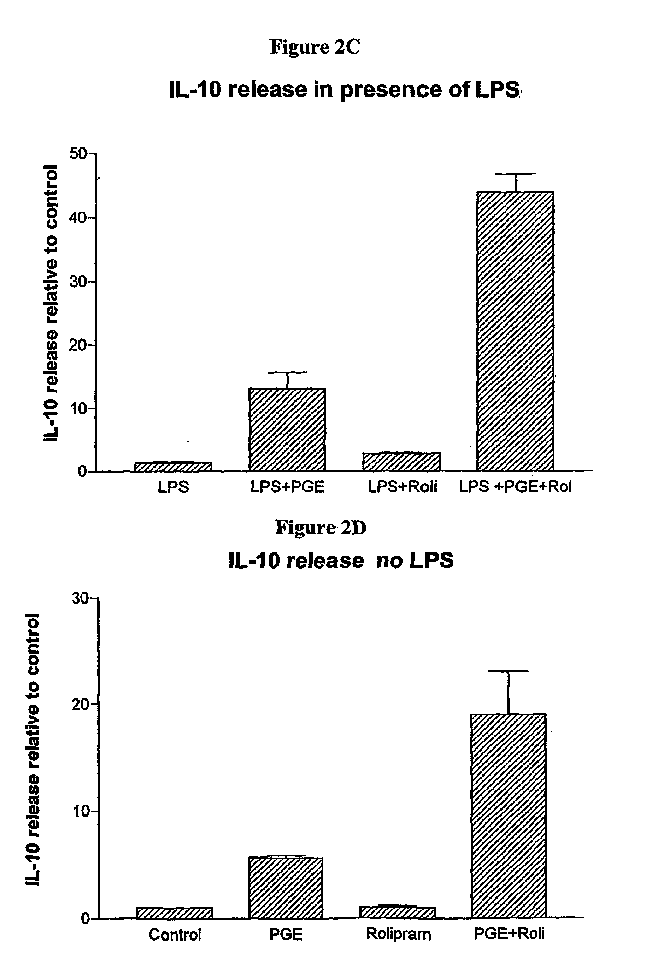

Figure 2A is a graph showing the effect of PGE and Rolipram on the production of IL-10 mRNA in U937 cells. Figure 2B is a graph showing the effect of LPS, PGE and Rolipram on the production of IL-10 mRNA in U937 cells. Figure 2C is a graph showing the effect of LPS, PGE and Rolipram on IL-10 release from U937 cells. Figure 2D is a graph showing the effect of PGE and Rolipram on IL-10 release from U937 cells.

### Figure 3

A graph showing the effect of 19 hydroxy PGE1 and 19 hydroxy PGE2 on the stimulation of IL-10 in the presence and absence of rolipram.

### Figure 4

A graph showing the effect of PGE1 and PGE2 on the stimulation of IL-10 in the presence and absence of rolipram.

### Figure 5

A graph showing the effect of PGE and 19 hydroxy PGE on the production of phosphodiesterase IV b mRNA in the presence and absence of rolipram.

### Example 1: Effect of the combination of PGE and rolipram on IL-10 and IL-12 production by U-937 (promonocyte) cells

U 937 (human monocyte cell line) cells were grown in RPMI (PAA Laboratories) medium with 10% fetal calf serum added (PAA Laboratories). Cells were treated with prostaglandin E 2 at 10⁻⁶ Molar or with Interferon-γ at 10 ng/ml for 24 hours. Rolipram at 1 µg/ml and indomethacin at 10 µM was present in all wells. Cells were pelleted and the mRNA was extracted with Tri reagent (Sigma, Poole, UK). Total RNA was obtained by addition of chloroform and subsequent isopropanol precipitation. RNA was reverse transcribed with reverse transcriptase (Applied Biosystems) and random hexamers (Applied Biosystems). Probes and primers for IL-10 and IL-12 (p35) were designed using Primer Express (Applied Biosystems) and were as follows:
IL-12 p35 primers 
CCACTCCAGACCCAGGAATG
TGTCTGGCCTTCTGGAGCAT
IL-12Probe
   TCCCATGCCTTCACCACTCCCAA
IL-10, primers 
CTACGGCGCTGTCATCGAT
TGGAGCTTATTAAAGGCATTCTTCA
IL-10 probe
   CTTCCCTGTGAAAACAAGAGCAAGGCC

Template was amplified in a Taqman 7700 machine for 40 cycles using FAM/TAMRA dyes on the probe. The Applied Biosystems Kit was used to amplify and detect ribosomal (18S) RNA as a control. After 40 cycles the Ct (related to cycle number at which signal appears) for the FAM and the 18S (VIC) were recorded and absolute relative quantitation was achieved using the formula 2^{-ΔΔ^{Ct}}.

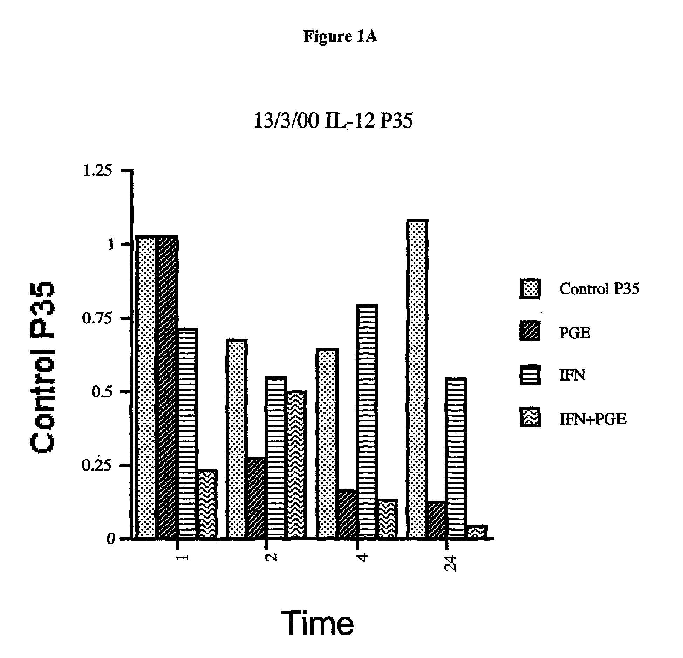

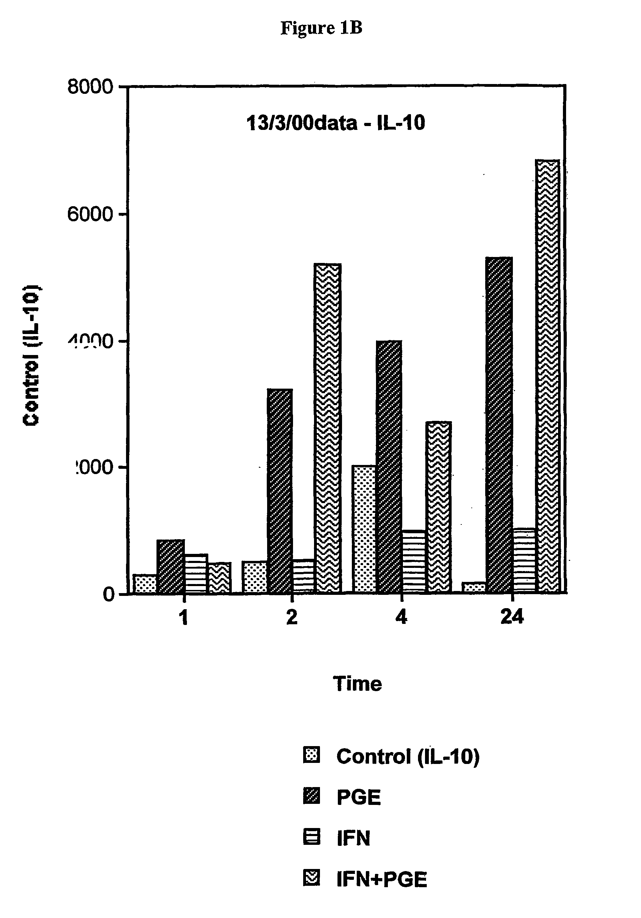

The results of this experiment are described in the legend to Figure 1. They show that there is a synergistic between a prostaglandin (PGE2) and a PDE inhibitor (rolipram) on the release of IL-10 from cells of the immune system and that there is a marked stimulation of IL-10 and inhibition of IL-12 in cells of the immune system when a prostaglandin (PGE2) and a PDE inhibitor (rolipram) are used in combination.

### Example 2: Stimulation of IL-10 production is achieved with or without LPS

U 937 cells were grown in RPMI (PAA Laboratories) medium with 10% fetal calf serum added (PAA Laboratories). 2 x 10⁶ cells per flask were treated with prostaglandin E₂ at 10⁻⁶ Molar or with Rolipram (4 x 10⁻⁶) for 24 hours. Medium was removed at 20 hours and analysed by ELISA. A capture antibody (Pharmingen) was coated onto 96 well plates and culture medium was added each well. A standard curve was created with recombinant IL-10 protein. After incubation and washing, a biotin labelled monoclonal antibody (Pharmingen) was added and following incubation and washing, peroxidase labelled streptavidin was added. After washing a tetramethyl benzidine substrate was added and colour developed in proportion to IL-10 in the original sample/standard. Colour was read using a plate photometer (Labsystems, Multiskan). Mean concentrations (N=3) in controls with no lipopolysaccharide (LPS) were 38.2pg/ml and in the presence of LPS (100nM) they were 43.9 prostaglandin/ml.

After the incubation (20 hours), cells were pelleted and the mRNA was extracted with Tri-reagent (Sigma, Poole, UK). Total RNA was obtained by addition of chloroform and subsequent isopropanol precipitation. RNA was reverse transcribed with reverse transcriptase (Applied Biosystems) and random hexamers (Applied Biosystems). Probes and primers for IL-10 and IL-12 (p35) were designed using Primer Express (Applied Biosystems) and were as follows:
IL-12 p35 primers 
CCACTCCAGACCCAGGAATG
TGTCTGGCCTTCTGGAGCAT
IL-12 probe
   TCCCATGCCTTCACCACTCCCAA
IL-10 primers 
CTACGGCGCTGTCATCGAT
TGGAGCTTATTAAAGGCATTCTTCA
IL-10 probe
   CTTCCCTGTGAAAACAAGAGCAAGGCC

Template was amplified in a Taqman 7700 machine for 40 cycles using FAM/TAMRA dyes on the probe. The Applied Biosystems kit was used to amplify and detect ribosomal (18S) RNA (using VIC/TAMRA dyes) as an internal control in the same reaction tube. After 40 cycles the Ct (related to cycle number at which signal appears) for the FAM and the 18S (VIC) were recorded and absolute relative quantitation was achieved using the formula 2^{-ΔΔCt} where Δ refers to the difference between the FAM and VIC signal related to an standard comparator included in each run.

### Example 3

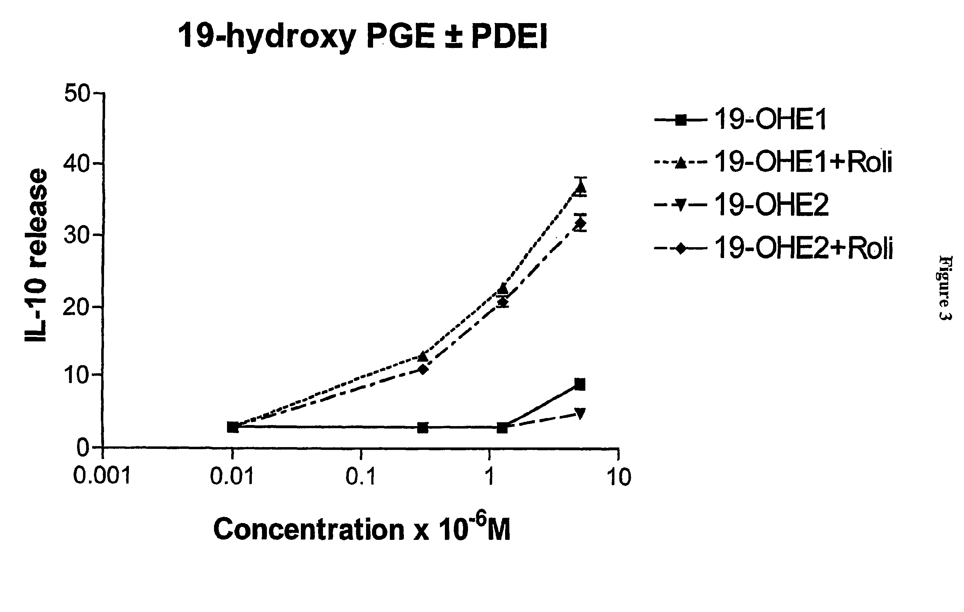

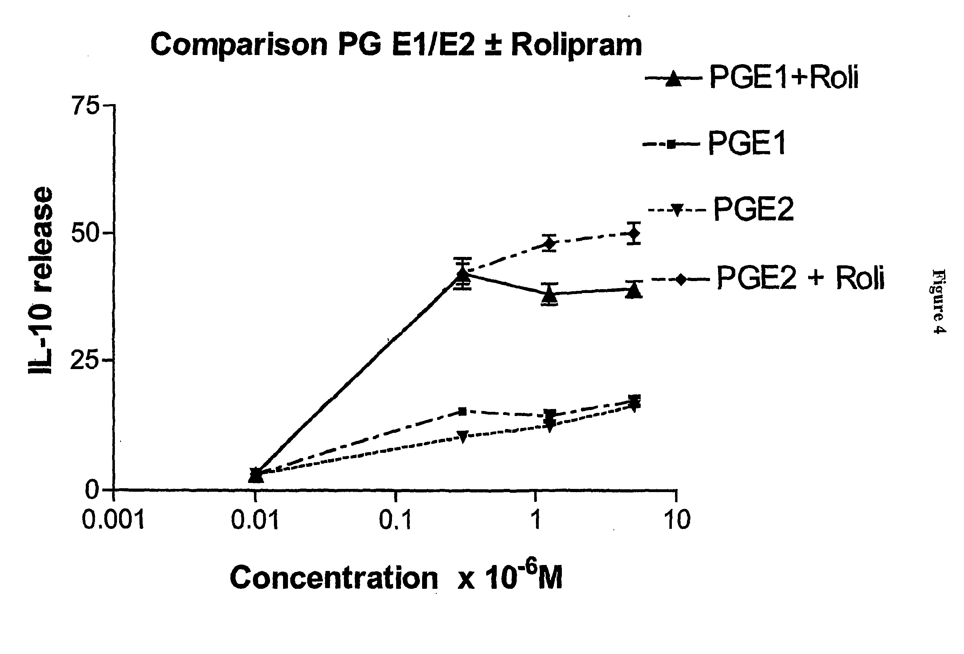

The effect of PGE1, PGE2, 19 hydroxy PGE1 and 19 hydroxy PGE2 on the stimulation of IL-10 in the presence and absence of rolipram was investigated as described above in Example 2. IL-10 levels were measured using an ELISA assay (R&D Ltd, Oxford). Measurements were taken in accordance with the manufacturer's instructions. Results are shown in Figures 3 and 4.

### Example 4

The mRNA for phosphodiesterase IV-b was measured as described in Example 2 above. mRNA was extracted after four hours of incubation. The concentration of the PGE was 1 x 10⁻⁶ and that of the 19-hydroxy PGE₂ was 5 x 10⁻⁶. The following primers and Taqman probe were used for quantitation of PDE IV b mRNA.
Forward
   CCTTCAGTAGCACCGGAATCA
Reverse
   CAAACAAACACACAGGCATGTAGTT
Probe
   AGCCTGCAGCCGCTCCAGCC

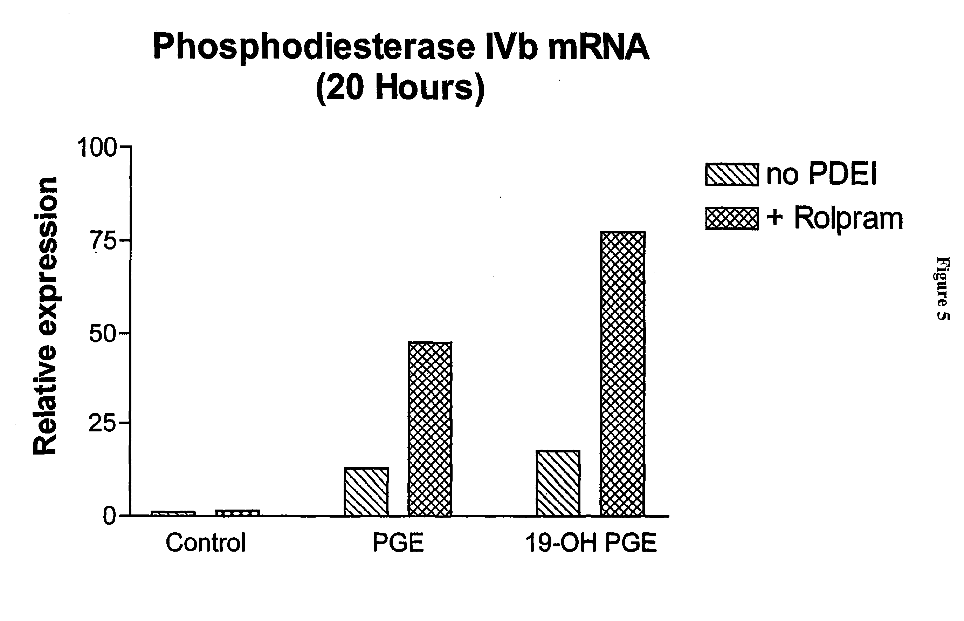

Results are shown in Figure 5. An increase in PDE activity follows both PGE and 19-hydroxy PGE application, which appears to be a direct negative feedback to reduce the effect of the stimulus. Use of a PGE and a type IV selective PDE inhibitor increases PDE message levels even further, but then the synthesised phosphodiesterase is nullified by the presence of the inhibitor.

### Example 5: Treatment of demyelinating disease

A patient with demyelinating disease is administered 800 µg misoprostol and 25 mg rolipram orally, daily.

## Claims

1. Use of a prostaglandin or agonist thereof that stimulates cAMP production in macrophages in the manufacture of a medicament for inducing tolerance to an antigen in a patient wherein the patient is administered a type IV selective PDE inhibitor and wherein the combination of the prostaglandin or agonist thereof and the type IV selective PDE inhibitor is used to induce tolerance to an antigen in the patient, with the exception of the treatment of rheumatoid arthritis.

2. Use of a type IV selective PDE inhibitor in the manufacture of a medicament for inducing tolerance to an antigen in a patient wherein the patient is administered a prostaglandin or agonist thereof that stimulates cAMP production in macrophages and wherein the combination of the prostaglandin or agonist thereof and the type IV selective PDE inhibitor is used to induce tolerance to an antigen in the patient.

3. Use of a combination of a prostaglandin or agonist thereof that stimulates cAMP production in macrophages and a type IV selective PDE inhibitor in the manufacture of a medicament for inducing tolerance to an antigen in a patient.

4. Use according to any one of Claims 1 to 3 wherein the PDE inhibitor selective for type IV PDE is any one of rolipram, CP80 633, CP102 995, CP76 593, Ro-20-1724, denbufylline, CDP 840, RP 73401 or RS 33793.

5. Use according to any one of Claims 1 to 4 wherein the prostaglandin or agonist thereof is any one of a prostaglandin E, such as prostaglandin E₂ or an analogue thereof, carboprost, dinoprostone, gemeprost, misoprostol, alprostadil, lamaprost, butaprost, 11-deoxy PGE1, AH23848, AH13205, 19-hydroxy PGE1 or 19-hydroxy PGE2 or agonist thereof.

6. Use according to any one of Claims 1 to 5 wherein the prostaglandin is a 19-hydroxy PGE.

7. Use according to any one of Claims 1 to 6 wherein the medicament is administered locally at a site where tolerance is required.

8. Use according to any one of Claims 1 to 7 wherein the medicament is formulated to be administered systemically.

9. Use according to Claim 8 wherein the medicament is formulated to be administered orally.

10. Use according to Claim 8 wherein the medicament is formulated as a suppository or capsule.

11. Use according to Claim 10 wherein the suppository or capsule has an enteric coating for release of the prostaglandin or agonist thereof and/or type IV selective PDE inhibitor in the bowel of the patient.

12. Use according to Claim 11 for treating inflammatory bowel disease.

13. Use according to any one of Claims 1 to 10 for combating a disease or condition associated with transplant rejection.

14. Use according to Claim 13 wherein the disease or condition associated with transplant rejection comprises graft versus host disease or host versus graft disease.

15. Use according to Claim 13 or 14 wherein the medicament is administered prior to the transplant.

16. Use according to any one of Claims 1 to 10 for treating an autoimmune disease other than rheumatoid arthritis.

17. Use according to Claim 16 wherein the autoimmune disease is selected from primary myxoedema, thyrotoxicosis, pernicious anaemia, autoimmune atrophic gastris, Addison's disease, IDDM, Goodpasture's syndrome, myasthenia gravis, sympathetic ophthalmia, MS, autoimmune haemolytic anaemia, idiopathic leucopenia, ulcerative colitis, dermatomyositis, scleroderma, mixed connective tissue disease, irritable bowel syndrome, SLE, Hashimoto's disease, thyroiditis, Behcet's disease, coeliac disease/dermatitis herpetifomis, and demyelinating disease.

18. Use according to any one of Claims 1 to 10 for treating an allergic disease or condition in the patient.

19. Use according to Claim 18 wherein the allergic disease or condition is allergic asthma.

20. Use according to Claim 19 wherein the prostaglandin is a 19-hydroxy PGE, and wherein the medicament is formulated as an aerosol for administration to the bronchial tree or lungs of the patient.

21. Use according to any one of Claims 1 to 20 wherein the tolerance to the antigen is to treat an aberrant or undesired immune response to the antigen in the patient.

22. Use according to Claim 21 wherein the aberrant or undesired immune response involves a deficiency in IL-10 production and/or an increase in IL-12 production.

23. A therapeutic system for use in inducing tolerance to an antigen in a patient, the system comprising a prostaglandin or agonist thereof that stimulates cAMP production in macrophages and a type IV selective PDE inhibitor.

24. A therapeutic system according to Claim 23 wherein the type IV selective PDE inhibitor is in a preparation for systemic administration.

25. A therapeutic system according to Claim 24 wherein the type IV selective PDE inhibitor is in a preparation for oral delivery.

26. A therapeutic system according to any one of Claims 23 to 25 wherein the prostaglandin or agonist is in a preparation for systemic administration, such as oral administration.

27. A therapeutic system according to any one of Claims 23 to 26 wherein the prostaglandin or agonist thereof or the type IV selective PDE inhibitor is as defined in any of claims 4 to 6.

28. A therapeutic system according to any one of Claims 23 to 27 for combating a disease or condition associated with transplant rejection.

29. A therapeutic system according to Claim 28 wherein the disease or condition associated with transplant rejection comprises graft versus host disease or host versus graft disease.

30. A therapeutic system according to Claim 28 or 29 wherein the prostaglandin or agonist thereof and/or PDE inhibitor arc for administration prior to the transplant.

31. A therapeutic system according to any one of Claims 23 to 27 for treating an autoimmune disease other than rheumatoid arthritis.

32. A therapeutic system according to Claim 31 wherein the autoimmune disease is selected from primary myxoedema, thyrotoxicosis, pernicious anaemia, autoimmune atrophic gastris, Addison's disease, IDDM, Goodpasture's syndrome, myasthenia gravis, sympathetic ophthalmia, MS, autoimmune haemolytic anaemia, idiopathic leucopenia, ulcerative colitis, dermatomyositis, scleroderma, mixed connective tissue disease, irritable bowel syndrome, SLE, Hashimoto's disease and thyroiditis, Behcet's disease, coeliac disease/dermatitis herpetiformis, and demyelinating disease.

33. A therapeutic system according to any one of Claims 23 to 27 for treating an allergic disease or condition in the patient.

34. A therapeutic system according to Claim 33 wherein the allergic disease or condition is allergic asthma.

35. A therapeutic system according to Claim 34 wherein the prostaglandin is a 19-hydroxy PGE, and wherein the 19-hydroxy PGE, and optionally the type IV selective PDE inhibitor, are for administration to the bronchial tree or lungs of the patient *via* an aerosol.

36. A therapeutic system according to any one of Claims 23 to 35, wherein the tolerance to the antigen is to treat an aberrant or undesired immune response to the antigen in the patient.

37. A therapeutic system according to Claim 36 wherein the aberrant or undesired immune response involves a deficiency in IL-10 production and/or an increase in IL-12 production.

## Patentansprüche

1. Verwendung eines Prostaglandins oder eines Agonisten davon, der die cAMP-Produktion in Makrophagen stimuliert, bei der Herstellung eines Medikaments zum Induzieren von Toleranz gegenüber einem Antigen bei einem Patienten, wobei dem Patienten ein selektiver PDE Typ IV-Inhibitor verabreicht wird und wobei die Kombination des Prostaglandins oder des Agonisten davon und des selektiven PDE Typ IV-Inhibitors verwendet wird, um Toleranz gegenüber einem Antigen in dem Patienten zu induzieren, mit der Ausnahme der Behandlung von rheumatischer Arthritis.

2. Verwendung eines selektiven PDE-Inhibitors bei der Herstellung eines Medikaments zum Induzieren von Toleranz gegenüber einem Antigen bei einem Patienten, wobei dem Patienten ein Prostaglandin oder ein Agonist davon verabreicht wird, der die cAMP-Produktion in Makrophagen stimuliert, und wobei die Kombination des Prostaglandins oder des Agonisten davon und des selektiven PDE Typ IV-Inhibitors verwendet wird, um Toleranz gegenüber einem Antigen bei dem Patienten zu induzieren.

3. Verwendung einer Kombination eines Prostaglandins oder eines Agonisten davon, der cAMP-Produktion in Makrophagen stimuliert, und eines selektiven PDE Typ IV-Inhibitors bei der Herstellung eines Medikaments zum Induzieren von Toleranz gegenüber einem Antigen bei einem Patienten.

4. Verwendung nach einem der Ansprüche 1 bis 3, wobei der PDE-Inhibitor, der selektiv für PDE vom Typ IV ist, Rolipram, CP80 633, CP102 995, CP76 593, Ro-20-1724, Denbufylin, CDP 840, RP 73401 oder RS 33793 ist.

5. Verwendung nach einem der Ansprüche 1 bis 4, wobei das Prostaglandin oder der Agonist davon Prostaglandin E, wie beispielsweise Prostaglandin E₂ oder ein Analogon davon, Carboprost, Dinoproston, Gemeprost, Misoprostol, Alprostatil, Lamaprost, Butaprost, 11-Deoxy-PGE1, AH23848, AH13205, 19-Hydroxy-PGE1 oder 19-Hydroxy-PGE2 oder ein Agonist davon ist.

6. Verwendung nach einem der Ansprüche 1 bis 5, wobei das Prostaglandin ein 19-Hydroxy-PGE ist.

7. Verwendung nach einem der Ansprüche 1 bis 6, wobei das Medikament lokal an dem Ort verabreicht wird, wo Toleranz erforderlich ist.

8. Verwendung nach einem der Ansprüche 1 bis 7, wobei das Medikament formuliert wird, um systemisch verabreicht zu werden.

9. Verwendung nach Anspruch 8, wobei das Medikament formuliert wird, um oral verabreicht zu werden.

10. Verwendung nach Anspruch 8, wobei das Medikament als Suppositorium oder Kapsel formuliert wird.

11. Verwendung nach Anspruch 10, wobei das Suppositorium oder die Kapsel eine magensaftresistente Beschichtung zum Freisetzen des Prostaglandins oder des Agonisten davon und/oder des selektiven PDE Typ IV-Inhibitors im Darm des Patienten aufweist.

12. Verwendung nach Anspruch 11 zur Behandlung von entzündlicher Darmerkrankung.

13. Verwendung nach einem der Ansprüche 1 bis 10 zur Bekämpfung einer Krankheit oder eines Zustands, die mit Transplantatabstoßung verbunden ist.

14. Verwendung nach Anspruch 13, wobei die Krankheit oder der Zustand, der mit Transplantatabstoßung verbunden ist, Transplantat-Wirt- oder Wirt-Transplantat-Reaktion umfasst.

15. Verwendung nach Anspruch 13 oder 14, wobei das Medikament vor dem Transplantat verabreicht wird.

16. Verwendung nach einem der Ansprüche 1 bis 10 zur Behandlung einer anderen Autoimmunerkrankung als rheumatischer Arthritis.

17. Verwendung nach Anspruch 16, wobei die Autoimmunerkrankung ausgewählt ist aus primärem Myxödem, Thyreotoxikose, krankhafter Anämie, autoimmuner atrophischer Gastritis, Addison-Krankheit, IDDM, Goodpasture-Syndrom, Myasthenia gravis, sympathetischer Ophthalmie, MS, autoimmuner hämolytischer Anämie, idiopathischer Leukopenie, ulcerativer Kolitis, Dermatomyositis, Sklerodermie, Mixed connective tissue disease, Reizdarmsyndrom, SLE, Hashimoto-Krankheit, Thyroiditis, Behcet-Krankheit, Zöliakie/Dermatitis Herpetiformis und demyelinisierender Krankheit.

18. Verwendung nach einem der Ansprüche 1 bis 10 zur Behandlung einer allergischen Erkrankung oder eines allergischen Zustands bei dem Patienten.

19. Verwendung nach Anspruch 18, wobei die allergische Erkrankung oder der allergische Zustand allergisches Asthma ist.

20. Verwendung nach Anspruch 19, wobei das Prostaglandin ein 19-Hydroxy-PGE ist und wobei das Medikament als ein Aerosol zur Verabreichung an die Bronchien oder die Lungen des Patienten formuliert wird.

21. Verwendung nach einem der Ansprüche 1 bis 20, wobei die Toleranz gegenüber dem Antigen vorgesehen ist, um eine gestörte oder unerwünschte Immunantwort auf das Antigen bei dem Patienten zu behandeln.

22. Verwendung nach Anspruch 21, wobei die gestörte oder unerwünschte Immunantwort einen Mangel an IL-10-Produktion und/oder einen Anstieg bei IL-12-Produktion einschließt.

23. Therapeutisches System zur Verwendung beim Induzieren von Toleranz gegenüber einem Antigen bei einem Patienten, wobei das System ein Prostaglandin oder einen Agonisten davon, der cAMP-Produktion in Makrophagen stimuliert, und einen selektiven PDE Typ IV-inhibitor aufweist.

24. Therapeutisches System nach Anspruch 23, wobei der selektive PDE Typ IV-Inhibitor in einer Präparation zur systemischen Verabreichung vorliegt.

25. Therapeutisches System nach Anspruch 24, wobei der selektive PDE Typ IV-Inhibitor in einer Präparation zur oralen Verabreichung vorliegt.

26. Therapeutisches System nach einem der Ansprüche 23 bis 25, wobei das Prostaglandin oder der Agonist in einer Präparation zur systemischen Verabreichung, wie beispielsweise zur oralen Verabreichung, vorliegt.

27. Therapeutisches System nach einem der Ansprüche 23 bis 26, wobei das Prostaglandin oder der Agonist davon oder der selektive PDE Typ IV-Inhibitor so ist, wie einem der Ansprüche 4 bis 6 definiert ist.

28. Therapeutisches System nach einem der Ansprüche 23 bis 27 zum Bekämpfen einer Krankheit oder eines Zustands, die/der mit Transplantatabstoßung verbunden ist.

29. Therapeutisches System nach Anspruch 28, wobei die Krankheit oder der Zustand, die/der mit Transplantatabstoßung verbunden ist, Transplantat-Wirt- oder Wirt-Transplantat-Reaktion umfasst.

30. Therapeutisches System nach Anspruch 28 oder 29, wobei das Prostaglandin oder der Agonist davon und/oder der PDE-Inhibitor zur Verabreichung vor dem Transplantat vorgesehen sind.

31. Therapeutisches System nach einem der Ansprüche 23 bis 27 zum Behandeln einer anderen Autoimmunerkrankung als rheumatische Arthritis.

32. Therapeutisches System nach Anspruch 31, wobei die Autoimmunerkrankung ausgewählt ist aus primärem Myxödem, Thyreotoxikose, krankhafter Anämie, autoimmuner atrophischer Gastritis, Addison-Krankheit, IDDM, Goodpasture-Syndrom, Myasthenia gravis, sympathetischer Ophthalmie, MS, autoimmuner hämolytischer Anämie, idiopathischer Leukopenie, ulcerativer Kolitis, Dermatomyositis, Sklerodermie, Mixed connective tissue disease, Reizdarmsyndrom, SLE, Hashimoto-Krankheit, Thyroiditis, Behcet-Krankheit, Zöliakie/Dermatitis Herpetiformis und demyelinisierender Krankheit.

33. Therapeutisches System nach einem der Ansprüche 23 bis 27 zur Behandlung einer allergischen Erkrankung oder eines allergischen Zustands bei dem Patienten.

34. Therapeutisches System nach Anspruch 33, wobei die allergische Erkrankung oder der allergische Zustand allergisches Asthma ist.

35. Therapeutisches System nach Anspruch 34, wobei das Prostaglandin ein 19-Hydroxy-PGE ist und wobei das 19-Hydroxy-PGE und optional der selektive PGE Typ IV-Inhibitor zur Verabreichung an die Bronchien oder Lungen des Patienten über ein Aerosol vorgesehen ist/sind.

36. Therapeutisches System nach einem der Ansprüche 23 bis 35, wobei die Toleranz gegenüber dem Antigen dazu dient, eine gestörte oder unerwünschte Immunantwort auf das Antigen bei dem Patienten zu behandeln.

37. Therapeutisches System nach Anspruch 36, wobei die gestörte oder unerwünschte Immunantwort einen Mangel an IL-10-Produktion und/oder einen Anstieg bei IL-12-Produktion einschließt.

## Revendications

1. Utilisation d'une prostaglandine ou d'un agoniste de celle-ci qui stimule la production d'AMPc dans les macrophages dans la fabrication d'un médicament pour induire une tolérance à un antigène chez un patient, dans laquelle on administre au patient un inhibiteur sélectif de la PDE de type IV et dans laquelle l'association de la prostaglandine ou de l'agoniste de celle-ci et de l'inhibiteur sélectif de la PDE de type IV est utilisée pour induire la tolérance à un antigène chez le patient, à l'exception du traitement de la polyarthrite rhumatoïde.

2. Utilisation d'un inhibiteur sélectif de la PDE de type IV dans la fabrication d'un médicament pour induire une tolérance à un antigène chez un patient, dans laquelle on administre au patient une prostaglandine ou un agoniste de celle-ci qui stimule la production d'AMPc dans les macrophages et dans laquelle l'association de la prostaglandine ou de l'agoniste de celle-ci et de l'inhibiteur sélectif de la PDE de type IV est utilisée pour induire une tolérance à un antigène chez le patient.

3. Utilisation d'une association d'une prostaglandine ou d'un agoniste de celle-ci qui stimule la production d'AMPc dans les macrophages et d'un inhibiteur sélectif de la PDE de type IV dans la fabrication d'un médicament pour induire une tolérance à un antigène chez un patient.

4. Utilisation selon l'une quelconque des revendications 1 à 3, dans laquelle l'inhibiteur de PDE sélectif pour la PDE de type IV est l'un quelconque parmi le rolipram, CP80 633, CP102 995, CP76 593, Ro-20-1724, la dembufylline, CDP 840, RP 73401 ou RS 33793.

5. Utilisation selon l'une quelconque des revendications 1 à 4, dans laquelle la prostaglandine ou l'agoniste de celle-ci sont l'un quelconque parmi la prostaglandine E, comme la prostaglandine E₂, ou un analogue de celle-ci, le carboprost, la dinoprostone, le géméprost, le misoprostol, l'alprostadil, le limaprost, la butaprost, le 11-désoxy-PGE1, AH23848, AH13205, la 19-hydroxy PGE1 ou la 19-hydroxy PGE2 ou un agoniste de ceux-ci.

6. Utilisation selon l'une quelconque des revendications 1 à 5, dans laquelle la prostaglandine est la 19-hydroxy PGE.

7. Utilisation selon l'une quelconque des revendications 1 à 6, dans laquelle le médicament est administré localement sur un site où une tolérance est requise.

8. Utilisation selon l'une quelconque des revendications 1 à 7, dans laquelle le médicament est formulé pour être administré par voie systémique.

9. Utilisation selon la revendication 8, dans laquelle le médicament est formulé pour être administré par voie orale.

10. Utilisation selon la revendication 8, dans laquelle le médicament est formulé sous forme de suppositoire ou de capsule.

11. Utilisation selon la revendication 10, dans laquelle le suppositoire ou la capsule a un enrobage entérique pour une libération de la prostaglandine ou de l'agoniste de celle-ci et/ou de l'inhibiteur sélectif de la PDE de type IV dans l'intestin du patient.

12. Utilisation selon la revendication 11, pour traiter une maladie intestinale inflammatoire.

13. Utilisation selon l'une quelconque des revendications 1 à 10, pour combattre une maladie ou un état associé(e) à un rejet de greffe.

14. Utilisation selon la revendication 13, dans laquelle la maladie ou l'état associé(e) à un rejet de greffe comprend la maladie du greffon contre l'hôte ou la maladie de l'hôte contre le greffon.

15. Utilisation selon la revendication 13 ou 14, dans laquelle le médicament est administré avant la greffe.

16. Utilisation selon l'une quelconque des revendications 1 à 10, pour traiter une maladie auto-immune autre que la polyarthrite rhumatoïde.

17. Utilisation selon la revendication 16, dans laquelle la maladie auto-immune est choisie parmi le myxoedème primitif, l'hyperthyroïdie, l'anémie pernicieuse, la gastrite atrophique auto-immune, la maladie d'Addison, le DID, le syndrome de Goodpasture, la myasthénie grave, l'ophtalmie sympathique, la SEP, l'anémie hémolytique auto-immune, la leucopénie idiopathique, la rectocolite hémorragique, la dermatomyosite, la sclérodermie, la connectivité mixte, le syndrome de l'intestin irritable, le LED, la maladie d'Hashimoto, la thyroïdite, la maladie de Behcet, la maladie coeliaque/dermatite herpétiforme et la maladie démyélinisante.

18. Utilisation selon l'une quelconque des revendications 1 à 10, pour traiter une maladie ou un état allergique chez le patient.

19. Utilisation selon la revendication 18, dans laquelle la maladie ou l'état allergique est l'asthme allergique.

20. Utilisation selon la revendication 19, dans laquelle la prostaglandine est une 19-hydroxy PGE et dans laquelle le médicament est formulé sous forme d'aérosol pour administration à l'arbre bronchique ou aux poumons du patient.

21. Utilisation selon l'une quelconque des revendications 1 à 20, dans laquelle la tolérance à l'antigène vise à traiter une réponse immunitaire aberrante ou indésirable contre l'antigène chez le patient.

22. Utilisation selon la revendication 21, dans laquelle la réponse immunitaire aberrante ou indésirable implique une déficience en production d'IL-10 et/ou une augmentation de la production d'IL-12.

23. Système thérapeutique à utiliser pour induire une tolérance à un antigène chez un patient, le système comprenant une prostaglandine ou un agoniste de celle-ci qui stimule la production d'AMPc dans les macrophages et un inhibiteur sélectif de la PDE de type IV.

24. Système thérapeutique selon la revendication 23, dans lequel l'inhibiteur sélectif de la PDE de type IV est dans une préparation pour administration systémique.

25. Système thérapeutique selon la revendication 24, dans lequel l'inhibiteur sélectif de la PDE de type IV est dans une préparation pour administration orale.

26. Système thérapeutique selon l'une quelconque des revendications 23 à 25, dans lequel la prostaglandine ou l'agoniste est dans une préparation pour administration systémique, comme l'administration orale.

27. Système thérapeutique selon l'une quelconque des revendications 23 à 26, dans lequel la prostaglandine ou l'antagoniste de celle-ci ou l'inhibiteur sélectif de la PDE de type IV est tel(le) que défini(e) dans l'une quelconque des revendications 4 à 6.

28. Système thérapeutique selon l'une quelconque des revendications 23 à 27, pour combattre une maladie ou un état associé(e) à un rejet de greffe.

29. Système thérapeutique selon la revendication 28, dans lequel la maladie ou l'état associé(e) au rejet de greffe comprend la maladie du greffon contre l'hôte ou la maladie de l'hôte contre le greffon.

30. Système thérapeutique selon la revendication 28 ou 29, dans lequel la prostaglandine ou son agoniste et/ou l'inhibiteur de la PDE sont destinés à être administrés avant la greffe.

31. Système thérapeutique selon l'une quelconque des revendications 23 à 27, pour traiter une maladie auto-immune autre que la polyarthrite rhumatoïde.

32. Système thérapeutique selon la revendication 31 dans lequel la maladie auto-immune est choisie parmi le myxoedème primitif, l'hyperthyroïdie, l'anémie pernicieuse, la gastrite atrophique auto-immune, la maladie d'Addison, le DID, le syndrome de Goodpasture, la myasthénie grave, l'ophtalmie sympathique, la SEP, l'anémie hémolytique auto-immune, la leucopénie idiopathique, la rectocolite hémorragique, la dermatomyosite, la sclérodermie, la connectivité mixte, le syndrome de l'intestin irritable, le LED, la maladie d'Hashimoto et la thyroïdite, la maladie de Behcet, la maladie coeliaque/dermatite herpétiforme et la maladie démyélinisante.

33. Système thérapeutique selon l'une quelconque des revendications 23 à 27, pour traiter une maladie ou un état allergique chez le patient.

34. Système thérapeutique selon la revendication 33 dans lequel la maladie ou l'état allergique est l'asthme allergique.

35. Système thérapeutique selon la revendication 34, dans lequel la prostaglandine est une 19-hydroxy PGE, et dans lequel la 19-hydroxy PGE, et éventuellement l'inhibiteur sélectif de la PDE de type IV, sont destinés à l'administration à l'arbre bronchique ou aux poumons du patient via un aérosol.

36. Système thérapeutique selon l'une quelconque des revendications 23 à 35, dans lequel la tolérance à l'antigène vise à traiter une réponse immunitaire aberrante ou indésirable contre l'antigène chez le patient.

37. Système thérapeutique selon la revendication 36, dans lequel la réponse immunitaire aberrante ou indésirable implique une déficience en production d'IL-10 et/ou une augmentation de la production d'IL-12.
